(19) Europäisches Patentamt
European Patent Office
Office européen des brevets

(11) Publication number: 0 309 056 A1

(12) # EUROPEAN PATENT APPLICATION

(21) Application number: 88202056.3

(22) Date of filing: 20.09.88

(51) Int. Cl.4: C07C 45/50 , C07C 47/02 , C07C 47/12

(30) Priority: 24.09.87 GB 8722460

(43) Date of publication of application: 29.03.89 Bulletin 89/13

(84) Designated Contracting States: AT BE CH DE ES FR GB GR IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Van Leeuwen, Petrus Wilhelmus Nicolaas Maria
Badhuisweg 3
NL-1031 CM Amsterdam(NL)
Inventor: Grotenhuis, Paul Alexander Maria
Badhuisweg 3
NL-1031 CM Amsterdam(NL)
Inventor: Goodall, Brian Leslie
Badhuisweg 3
NL-1031 CM Amsterdam(NL)

(74) Representative: Aalbers, Onno et al
P.O. Box 302
NL-2501 CH The Hague(NL)

(54) **Process for the hydroformylation of olefins.**

(57) A process for the preparation of a linear mono-or di-aldehyde, which comprises reacting an alpha-olefin or an alpha, omega-diolefin with carbon monoxide and hydrogen in the presence of a catalyst system comprising a rhodium compound and a ligand selected from bis(diphenylphosphinoethyl)ether and derivatives thereof in which one or more of the phenyl groups is or are substituted by lower alkyl, lower alkoxy, halogen, carboxy or cyano.

EP 0 309 056 A1

# PROCESS FOR THE HYDROFORMYLATION OF OLEFINS

The present invention relates to a process for the preparation of linear aldehydes by the hydroformylation of alpha-olefins. In particular it relates to a process in which a catalyst system comprising a rhodium compound and a multidentate phosphorus-containing ligand is used.

Several hydroformylation processes using a catalyst system comprising a rhodium compound and a multidentate phosphorus-containing ligand are known.

For example US 4,595,753, US 4,593,141 and US 4,668,809 describe processes for the production of aldehydes from olefins using certain specific classes of catalysts containing two diphenylphosphino residues. More particularly in table 15 (columns 77 and 78) of US 4,595,753 reference is made to bis-(diphenylphosphinopropyl)ether in the hydroformylation of 1-butene. The selectivity to the linear aldehyde product is 73.3%.

EP-A-0033554 discloses a process for the preparation of linear aldehydes by the hydroformylation of conjugated dienes. In this process a catalyst is used which comprises a complex of rhodium with a polydentate ligand containing trivalent phosphorus atoms bearing substituted or unsubstituted aryl groups and in which ligand any of two phosphorus atoms are separated by a chain of two carbon atoms.

There remains, however, a need for a process for the hydroformylation of alpha-olefins and alpha, omega-diolefins which affords linear aldehydes with high selectivity and low double-bond isomerisation.

The present invention provides a process for the preparation of a linear mono- or di-aldehyde, which comprises reacting an alpha-olefin or an alpha, omega-diolefin with carbon monoxide and hydrogen in the presence of a catalyst system comprising a rhodium compound and a ligand selected from bis-(diphenylphosphinoethyl)ether and derivatives thereof in which one or more of the phenyl groups is or are substituted by lower alkyl, lower alkoxy, halogen, carboxy or cyano. Preferably the ligand is bis-(diphenylphosphinoethyl)ether.

Preferably the rhodium compound is selected from soluble salts, such as rhodium (I) chloride and rhodium (I) acetate, and rhodium complexes such a 1,5-cyclooctadiene rhodium (I) acetate, 1,5-cyclooctadiene rhodium (I) acetoacetate, acetylacetonate (bis ethylene) rhodium (I), and acetylacetonatedicarbonyl rhodium (I).

Since soluble rhodium compounds which contain halogen atoms may cause corrosion in the hydroformylation equipment, such compounds should preferably not be used.

The molar ratio of rhodium compound to ligand may conveniently be in the range of from 1:2 to 1:100 and more preferably in the range from 1:10 to 1:50.

The molar ratio of rhodium compound to olefin may conveniently be in the range of from 1:5,000 to 1:100,000 and more preferably from 1:20,000 to 1:50,000.

In the process according to the invention the molar ratio of carbon monoxide to hydrogen may conveniently be in the range of from 3:1 to 1:3 and is preferably in the range of from 1.5:1 to 1:1.5.

Most conveniently the ratio is 1:1 since there will then be no need to bleed off excess carbon monoxide or hydrogen from the reaction vessel.

It will be appreciated that carbon monoxide-hydrogen mixtures can be used as such or may be diluted by an inert gas such as nitrogen or helium.

The temperature at which the process is effected is preferably in the range of from 65-100 °C.

The pressure under which the process is effected is conveniently in the range of from 1 to 5 bar, more preferably in the range 1.7 to 3.5 bar.

The process is conveniently effected in the presence of a solvent. As suitable solvents amides may be used such as dimethylformamide, dimethylacetamide or N-methylpyrrolidone; nitriles e.g. acetonitrile; ethers e.g. dioxane or tetrahydrofuran,. alcohols e.g. methanol, ethanol or propanol; and esters e.g. ethyl acetate. Preferably N,N-dimethylacetamide or N-methylpyrrolidone is used.

The process according to the invention may, when starting from diolefins, afford a mixture containing mono- and dialdehydes. It will be appreciated that the amount of monaldehyde in the mixture depends upon the reaction time until the reaction is stopped, e.g. by cooling.

Dialdehydes may be isolated from the mixture by fractional distillation or isolated in the form of dihemiacetal derived from a saturated primary alcohol (e.g. 1-hexanol) by crystallization and filtration.

Bis(diphenylphosphinoethyl)ether may be prepared according to methods described in Inorganica Chimica Acta, 97 (1985) 143-150.

The linearity of the mono- and dialdehydes prepared by the process according to the invention is high, whereas the isomerization of the starting olefins is low. This is both surprising and advantageous.

The following examples illustrate the invention.

Example 1

Preparation of undecenal and 1,12-dodecanedial by hydroformylation of 1,9-decadiene.

A 300 ml stainless steel autoclave equipped with a mechanical stirrer, a gas inlet tube, a thermocouple and a manometer, was charged with 1,9-decadiene (1.0 mol), N,N-dimethylacetamide (25 ml), bis-(diphenylphosphinoethyl)ether (0.5 mmol) and acetyl acetonato bis(ethylene) rhodium (I) while the molar ratio of the ligand and rhodium was 25 and the molar ratio between ligand and 1,9-decadiene was 1:2000.

The autoclave was flushed with an equimolar mixture of hydrogen and carbon monoxide and then pressurised to 2 bar.

The reaction mixture was heated up to 75 °C whilst maintaining the pressure at 2 bar. After 4 hours reaction time, the mixture was allowed to cool to room temperature. Decadiene conversion and the product composition have been indicated in table 1, together with the results from several further experiments in which the relative amount of rhodium, ligand and 1,9-decadiene were varied.

TABLE 1

Hydroformylation of 1,9-decadiene

Solvent :N,N-dimethyl acetamide

total pressure :2 bar (hydrogen/carbon monoxide ratio = 1/1)

| Exp. | Molar ratio | | | t | Analysis %w | | | | | | lin. mono aldehyde | % isomerization | |
|---|---|---|---|---|---|---|---|---|---|---|---|---|---|
| | Rh | Ligand | $C_{10}^{==}$ | hrs | 1,9 $C_{10}^{==}$ | 1,8 $C_{10}^{==}$ | α+β iso | α-n | β-n | dial | | $C_{10}^{==}$ * | mono-aldehyde |
| 1 | 1 | 25 | 50,000 | 4 | 58.1 | 0.5 | 2.9 | 34.4 | 0.2 | 3.9 | 92.2 | 0.8 | 0.6 |
| 2 | 1 | 24.5 | 49,835 | 4.00 | 43.6 | 1.0 | 2.8 | 38.0 | 0.5 | 14.0 | 93.2 | 2.4 | 1.3 |
| 3 | 1 | 93.0 | 46,835 | 6.75 | 61.2 | 0.6 | 2.4 | 29.1 | 0.4 | 6.3 | 92.3 | 1.0 | 1.3 |
| 4 | 1 | 24.7 | 50,301 | 4.20 | 45.6 | 1.0 | 2.7 | 37.6 | 0.5 | 12.3 | 93.5 | 2.1 | 1.3 |

TABLE 1 (Continued)

Hydroformylation of 1,9-decadiene

| Exp. | Molar ratio | | | t | Analysis %w | | | | | | lin. mono aldehyde | % isomerization | |
|---|---|---|---|---|---|---|---|---|---|---|---|---|---|
| | Rh | Ligand | $C_{10}^{==}$ | hrs | 1,9 $C_{10}^{==}$ | 1,8 $C_{10}^{==}$ | α+β iso | α-n | β-n | dial | | $C_{10}^{==}$ * | mono-aldehyde |
| 5 | 1 | 9.8 | 49,940 | 4.00 | 49.8 | 1.0 | 2.4 | 35.6 | 0.4 | 10.8 | 93.6 | 2.0 | 1.1 |
| 6 | 1 | 5.0 | 48,000 | 4.25 | 46.5 | 1.1 | 2.6 | 36.7 | 0.4 | 12.6 | 93.3 | 2.3 | 1.3 |
| 7 | 1 | 1.1 | 49,755 | 4.00 | 59.9 | 2.9 | 2.9 | 27.8 | 0.7 | 5.7 | 90.7 | 4.6 | 2.5 |

*% isomerization $C_{10}^{==}$ $$\frac{\%\ 1,8\ C_{10}^{==}}{\%\ 1,8 + 1,9\ C_{10}^{==}} \cdot 100\%$$

% isomerization undecenal $$\frac{\%\ \beta\text{-n}}{\%\ \alpha\text{-n} + \%\ \beta\text{-n}} \cdot 100$$

α-n= 10-undecenal

β-n= 9-undecenal

α + β iso= 2-methyl-9-decenal and 2-methyl-9-decenal

Ligand (1) $\phi_2P-CH_2-CH_2-O-CH_2-CH_2-P_2\phi$

Example 2

Preparation of undecanal by hydroformylation of 1-decene

In about the same way as described under example 1, experiments were carried out using four different ligands in a molar ratio between rhodium compound and ligand of 1:25 and an olefin/-rhodium ratio of 50,000. The total reaction pressure was 2 bar., the reaction time 4 hours and the reaction temperature 75° C. The ligands were compounds according to the formulae.

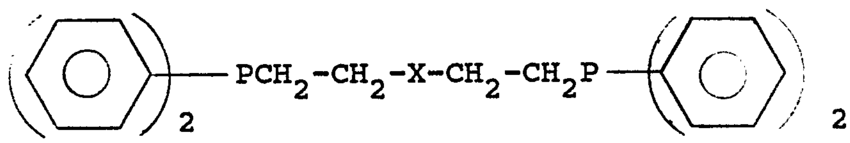

I wherein X = 0 (ligand of the present invention)
II wherein X = $CH_2$
III wherein X = N-$CH_3$ and

IV 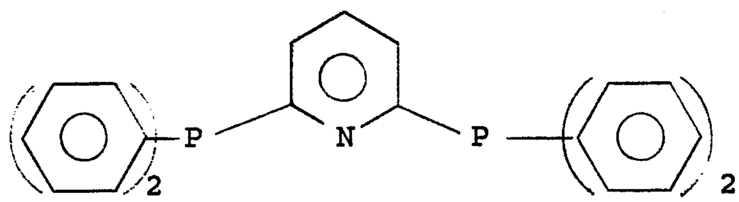

The obtained results have been summarized in table 2.

**Table 2**

| Exp. | ligand | conversion | selectivity | isomerization |
|---|---|---|---|---|
| 1 | I | 37% | 92.2 | 1.4 |
| 2 | II | 20 | 84 | 4% |
| 3 | III | none | | |
| 4 | IV | 5% | 80 | 4.2% |

Example 3

Preparation of 6-heptenal by hydroformylation of 1,5-hexadiene

In about the same way as described under example 1, experiments were carried out starting from 1,5-hexadiene using bis-(diphenylphosphinoethyl) ether as ligand under different reaction conditions.

The obtained results are summarized in table 3.

## Table 3
## Hydroformylation of hexadiene

| Exp | Molar ratio | | | T °C | P bar | Selectivity | Isomerization |
|---|---|---|---|---|---|---|---|
| | Rh | Ligand | $C_6^{==}$ | | | | |
| 1 | 1 | 10 | 25000 | 75 | 3.0 | 89 | 2.0 |
| 2 | 1 | 25 | 25000 | 75 | 3.0 | 92 | 1.0 |
| 3 | 1 | 50 | 25000 | 75 | 3.0 | 94 | 1.0 |

**Claims**

1. A process for the preparation of a linear mono-or di-aldehyde, which comprises reacting an alpha-olefin or an alpha, omega-diolefin, with carbon monoxide and hydrogen in the presence of a catalyst system comprising a rhodium compound and a ligand selected from bis(diphenylphosphinoethyl)ether and derivatives thereof in which one or more of the phenyl groups is or are substituted by lower alkyl, lower alkoxy, halogen, carboxy or cyano.

2. A process as claimed in claim 1, in which the ligand is bis(diphenylphosphinoethyl)ether.

3. A process as claimed in claim 1 or claim 2, in which the rhodium compound is selected from rhodium(I)chloride, rhodium(I) acetate, 1,5-cyclooctadiene rhodium(I)acetoacetate, acetylacetonate (bis-ethylene) rhodium(I) and acetylacetonatodicarbonyl rhodium (I).

4. A process as claimed in any one of the claims 1 to 3, in which the molar ratio of rhodium compound to ligand is in the range of from 1:10 to 1:50.

5. A process as claimed in any of the claims 1 to 4, in which the molar ratio of rhodium compound to olefin is in the range of from 1:20,000 to 1:50,000.

6. A process as claimed in any one of the claims 1 to 5, in which the molar ratio of carbon monoxide to hydrogen is in the range from 1.5:1 to 1:1.5.

7. A process as claimed in any one of the claims 1 to 6, in which the temperature is in the range of from 65-100 °C.

8. A process as claimed on any one of the claims 1 to 7, in which the pressure is in the range of from 1.7 to 3.5 bar.

9. A process as claimed in any one of the claims 1 to 8, in which a solvent is used selected from N,N-dimethylacetamide and N-methylpyrrolidone.

European Patent Office

# EUROPEAN SEARCH REPORT

Application number: EP 88202056.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US - A - 4 595 753 (ALEXIS A. OSWALD et al.) * Abstract; example 31, table 15 - no. 12 * -- | 1 | C 07 C 45/50<br>C 07 C 47/02<br>C 07 C 47/12 |
| D,X | US - A - 4 668 809 (ALEXIS A. OSWALD et al.) * Abstract; example 31, table 15 - no. 12 * -- | 1 | |
| A | US - A - 4 390 729 (ALEXIS A. OSWALD) * Claim 1 * -- | 1 | |
| A | US - A - 4 229 381 (IKUEI OGATA et al.) * Claim 1 * -- | 1 | **TECHNICAL FIELDS SEARCHED (Int. Cl.4)** |
| A | DE - A1 - 3 030 108 (KURARAY CO.) * Claim 1 * -- | 1 | C 07 C 45/00<br>C 07 C 47/00 |
| A | US - A - 4 215 077 (MITSUO MATSUMOTO et al.) * Abstract * -- | 1 | |
| A | GB - A - 2 185 740 (SHELL INTERNATIONAL RESEARCH) * Abstract * ---- | 1 | |

The present search report has been drawn up for all claims

| Place of search | Date of completion of the search | Examiner |
|---|---|---|
| VIENNA | 13-12-1988 | REIF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document